# EUROPÄISCHE PATENTSCHRIFT

(11) **EP 1 101 483 B1**
(45) Veröffentlichungstag und Bekanntmachung des Hinweises auf die Patenterteilung: **01.06.2005**
(21) Anmeldenummer: 00124909.3
(22) Anmeldetag: 15.11.2000
(51) Int. Cl.: A61J 1/05, A61M 1/16, A61J 1/00, A61M 1/28

(54) **Mehrkammerbehälter, mit Glucosekonzentratkompartiment und Salzsäurekonzentratkompartiment**
Multi compartment bag having one compartment for glucose concentrate and one compartment for hydrochloric acid
Sac compartimenté ayant un compartiment pour concentré de glucose et un compartiment pour acide chlorhydrique

(30) Priorität: 18.11.1999 DE 19955578
(43) Veröffentlichungstag der Anmeldung: 23.05.2001
(73) Patentinhaber: Fresenius Medical Care Deutschland GmbH, 61352 Bad Homburg v.d.H. (DE)
(72) Erfinder: Knerr, Thomas, Dr., 66606 St. Wendel (DE); Pott, Harald, 66557 Illingen (DE)
(74) Vertreter: Laufhütte, Dieter, Dr.-Ing.

(56) Entgegenhaltungen:
- WO-A-93/09820
- WO-A-93/24108
- DE-A- 4 410 876

## Beschreibung

Die Erfindung betrifft einen Mehrkammerbehälter mit mindestens zwei Kompartimenten, zur Aufbewahrung von Dialysekonzentraten. Die Dialyseflüssigkeit, die durch Verdünnung von Konzentraten und Vermischen mit weiteren Substanzen hergestellt wird, dient dabei zur Aufnahme von toxischen Substanzen bei nierenkranken Menschen.

Im Stand der Technik sind mehrere Mehrkammerbehälter bekannt, die verschiedene Lösungen in den Kompartimenten beinhalten. Diese Lösungen sollten zwar gemischt verabreicht werden, jedoch sind die Lösungen lagerinkompatibel. Daher sind die einzelnen Kompartimente dieser Mehrkammerbehälter bis kurz vor der Applikation der Lösungen getrennt. Zwischen den Kompartimenten ist eine Flüssigkeitsverbindung vorgesehen, die direkt vor der Anwendung geöffnet wird, damit die Flüssigkeiten gemischt werden können.

Die Fluidverbindung kann im Falle von Mehrkammerbehälter ein brechbarer Konnektor sein, oder eine peelfähige Schweißnaht, wie im Patent DE 44 10 876 C der Anmelderin beschrieben. Durch Druck auf den Behälter öffnet sich dabei die peelfähige Naht und die Lösungen sind in einem einzigen Raum ohne Totvolumen leicht miteinander mischbar. Eine typische Anwendung dieser Mehrkompartimentbehälter ist im Bereich der Nieren-Dialyse, speziell der Peritonealdialyse zu finden, wo Lösungen als Aufnahmemedium für die Giftstoffe des Körpers in Ersatz für eine nicht funktionsfähige Niere fungieren.

Diese Lösungen beinhalten stets ein Puffersystem, das mit einer Säure in den physiologischen Bereich gebracht wird, sowie eine Elektrolytlösung. Da der Puffer des Blutes Bicarbonat ist, wird heute meist auch dieser Puffer für Dialyselösungen gewählt. Als Säure ist Essigsäure weit verbreitet, da diese im Citratcyclus abgebaut werden kann. Um eine Veränderung der lebensnotwendigen Elektrolytkonzentrationen im Körper der Patienten zu vermeiden, sind den Lösungen Calcium-, Magnesium- und Kaliumionen meist in Form ihrer Chloride zugesetzt, sowie eine im Vergleich dazu relativ hohe Konzentration an Kochsalz oder NaCI. Als Osmotikum, um den Druck zu erzeugen, der den Stoffaustausch bewirkt oder zumindest beschleunigt, wird meist Glucose verwendet.

Die getrennte Lagerung der einzelnen Bestandteile aus mangelnden Kompatibilitätsgründen ist bereits im Stand der Technik beschrieben. Das Patent DE 39 17 251 C zeigt beispielsweise ein Zweikammerbehältersystem zur Herstellung einer Peritonealdialyselösung, wobei in einer Kammer eine saure Konzentratlösung mit zumindest Calciumionen aufbewahrt wird, in einer zweiten Kammer eine Bicarbonatlösung. Im vorliegenden Fall besteht die Gefahr, daß sich bei gemeinsamer Lagerung der Komponenten schwerlösliches Calciumcarbonat bildet und in der Lösung ausfällt. Daher wird Calcium und Bicarbonat getrennt aufbewahrt und erst kurz vor Applikation der Lösung in den Bauchraum des Patienten gemischt.

Aus o.g. Gründen ist man dazu übergegangen, das Bicarbonat stets getrennt zu den anderen Bestandteilen einer Dialysierlösung aufzubewahren. Um Transport- und Lagerkosten einzusparen, empfiehlt es sich auch, die Bestandteile in möglichst konzentrierter Form am Behandlungsort bereitzustellen. Daher ist in der EP 0 278 100 A vorgeschlagen worden, das Bicarbonat als Festsubstanz in einer von allen anderen Bestandteilen getrennten Kartusche vorzulegen. Auch das NaCI bietet sich als leichtlösliches Salz an, in fester Form und als getrennte Einheit vorzulegen.

In diesen Fällen ist es notwendig, die Pulver getrennt aufzubewahren, da durch die unterschiedlichen Löslichkeitsprodukte im Laufe des Lösevorgangs Konzentrationsschwankungen auftreten würden. Vorteilhaft wäre es natürlich, auch die Säure, die Elektrolyte und die Glucose in fester Form vorzulegen, doch stößt dies in der Praxis auf Löslichkeits- und Dosierprobleme. Zum einen sind die Elektrolyte zu hygroskopisch, zum anderen liegen Säuren, wie die üblicherweise verwendete Essigsäure, ohnehin bei Raumtemperatur als Flüssigkeit vor. Aus diesen Gründen ist es bislang üblich, diese Bestandteile als hochkonzentrierte Flüssigkeiten bereit zu stellen, um die Transportkosten zu senken.

Die Trennung auch dieser Bestandteile ist - die Glucose betreffend - notwendig, da derartige Lösungen meist hitzesterilisiert werden, wodurch jedoch ein Abbau der Glucose hervorgerufen werden kann. In der WO 93/09820 wird daher vorgeschlagen, die Glucose in einem separat gehaltenen Behälterteil unterzubringen, wobei der Glucosegehalt mindestens 10% beträgt und der pH-Wert um 3,5 ist, also die Säure enthalten ist. Durch die Säure soll der Abbau der Glucose bei der Sterilisation unterdrückt werden. In dem zweiten Kompartiment befinden sich die Elektrolyte und ein Lactatpuffer.

Aus der WO-A-9 309 820 ist ein Mehrkammerbehälter zur Bereitstellung einer sterilen und glukosehaltigen medizinischen Lösung bekannt, wobei das eine Kompartiment ein glucosehaltiges, saures Konzentrat und das andere Kompartiment mit einer elektrolythaltigen Lösung mit einem pH-Wert von 7-9 gefüllt ist. Zur Vermischung des Inhalts der beiden Kompartimente ist eine aufbrechbare Trennlinie vorgesehen.

Im Laufe der ärztlichen Praxis hat sich herausgestellt, daß Essigsäure und andere im Citratcyclus abbaubare Säuren klinische Nebenwirkungen zeigen, beispielsweise eine Acidose hervorrufen können. Aus diesem Grunde geht man dazu über, eine acetatfreie Dialyse zu fordern. Eine physiologisch verträgliche Säure, die früher in der Dialyse eingesetzt wurde, ist Salzsäure. Die Salzsäure, als starke Säure, wurde mit Einzug des Bicarbonates als Puffersystem verdrängt, da bei einer Mischung leicht Kohlendioxid entsteht. Des weiteren hat Salzsäure den Nachteil, nicht hitzesterilisierbar zu sein, da HCI als Gas sich zu leicht verflüchtigt und stark korrodierend wirkt.

Aufgabe der vorliegenden Erfindung ist es, ein Dialysekonzentrat bereit zu stellen, das eine acetatfreie Dialyse erlaubt. Die Aufgabe wird durch die im ersten Anspruch aufgezeigten Merkmale gelöst.

Der erfindungsgemäße Mehrkammerbehälter besteht aus flexiblem, biokompatiblem und durchsichtigem Material, vorzugsweise aus Polyolefinen und weist ein erstes Kompartiment auf, das ein Glucosekonzentrat enthält und ein zweites Kompartment, das ein Salzsäurekonzentrat enthält inklusive Calcium-, Magnesium- und Kaliumionen, sowie Chloriden. Die beiden Kompartimente sind durch eine peelbare Trennaht getrennt. Kurz vor Verwendung wird die Peelnaht durch Druck auf die beiden Kammern geöffnet und die beiden Konzentrate gemischt. Denkbar ist auch eine Konnektion zwischen den beiden Kompartimenten, beispielsweise ein mit einer Sollbruchstelle versehener Konnektor, der nach Aufbrechen einen Flüssigkeitsweg öffnet.

Um eine acetatfreie Dialyse zu ermöglichen, ist Salzsäure als Säurebestandteil vorgesehen. Da Salzsäure eine starke Säure ist, bewirkt eine Langzeitlagerung mit Glucose zusammen eine Zersetzung der Lösung. Daher ist eine Lagerung zusammen mit Glucose nicht möglich und die Salzsäure wird erfindungsgemäß in einem zweiten Kompartiment zur Verfügung gestellt. Weder kann die nicht angesäuerte Glucoselösung - wie in oben genannter WO 93/09820 beschrieben - einer Hitzsterilisation unterzogen werden, noch die gasförmige Salzsäure. Daher wird die Lösung zwar steril hergestellt und unter möglichst sterilen Bedingungen abgefüllt, jedoch der bereitgestellte Behälter keiner Sterilisation mehr unterzogen.

Gemäß der vorliegenden Erfindung enthält das Säurekompartiment auch die Elektrolyte Calcium, Magnesium und Kalium. Optional kann sowohl dem Säurekompartiment als auch dem Glucosekonzentrat Kochsalz, NaCI, beigefügt werden. Die Transportkosten können aufgrund der hohen Konzentration und damit des geringen Volumens erheblich gesenkt werden. Auch die Vorratshaltung in der Klinik ist extrem vereinfacht. Darüber hinaus ist es logistisch aufgrund des geringen Lagerbedarfs möglich, verschiedene Konzentratbehälter zu bevorraten und damit ein für die Patienten individual eingestelltes Elektrolytkonzentrat vorzugeben.

Typischerweise ist ein Konzentrat 70-200fach konzentriert, vorzugsweise 125fach. Um die für die Dialyse üblichen Konzentrationen zu erreichen, ist daher bei einem 125-fach Konzentrat folgende Zusammensetzung beispielhaft einzustellen:

| | | | |
|---|---|---|---|
| Lösung A | Natriumchlorid | 1 250mM | |
| | Kaliumchlorid | 500mM | |
| | Calciumchlorid | 375mM | |
| | Salzsäure | 750mM | pH≤1 |
| | | | |
| Lösung B | Natriumchlorid2500 mM | | |
| | Glucose | 250g/l | pH=3 |

Jedes Kompartiment ist dabei vorzugsweise gleich groß und enthält eine Menge von 0,75 Liter Konzentrat. Jedoch kann auch vorgesehen werden, das Mischungsverhältnis von 1 zu 3 bis 3 zu 1 zu variieren und dann zweckmäßigerweise den Mehrkammerbehälter auch asymmetrisch auszugestalten.

Zur Verdeutlichung sind nachfolgend beispielhaft verschiedene Mischungsverhältnisse, sowie Konzentrationen tabellarisch aufgeführt. Unabhängig davon wie die Konzentrationen oder das Mischungsverhältnis gewählt werden, sollten folgende Konzentrationsbereiche in der fertigen Lösung eingehalten werden.

**Tabelle 1:**

| Konzentrationsbereiche in der fertigen Lösung. | |
|---|---|
| NaCl | 0-20mM |
| KCI | 0-4mM |
| CaCl₂ | 1-2mM |
| MgCl₂ | 0,25-0,75mM |
| HCl | 2-4mM |
| Glucose | 0,5-2g/l (=3-11mM) |

Somit bewegen sich die Konzentrationen in den einzelnen Kompartimenten zwischen den Bereichen:

**Tabelle 2:**

| Konzentrationsbereiche in einzelnen Kompartimenten. | |
|---|---|
| NaCl | 0 - 4M |
| KCI | 0 - 800mM |
| CaCl₂ | 70 - 400mM |
| MgCl₂ | 17,5 - 200mM |
| HCl | 140 - 800mM |
| Glucose | 100-400g/l (=0,6-2,2M) |

Erfindungsgemäß ist weiterhin vorgesehen, daß die beiden Konzentrate innerhalb des Vorlagebehälters, also des Mehrkammerbehälters, gemischt werden und damit nur einen Auslaß benötigen. Damit sich der Auslaß nicht vorzeitig öffnet, ist auch dieser mit einem lösbaren Verschluß versehen. Dies kann beispielsweise ein Konnektor mit einer Sollbruchstelle sein, wobei nach Abbrechen der Sollbruchstelle ein Flüssigkeitsweg geöffnet wird. Vorzugsweise ist jedoch vorgesehen, auch diesen Verschluß mit einer Trennaht auszuführen. Damit ergibt sich ein Dreikammerbehälter, dessen erstes Kompartiment ein Glucosekonzentrat enthält, das zweite Kompartiment ein Salzsäurekonzentrat und das dritte Kompartiment im wesentlichen leer ist und in seinem Randbereich einen Auslaß besitzt.

Die dritte Kammer kann gebildet werden, indem die Peelnaht zwischen den beiden Kompartimenten sich im unteren Ende in zwei Äste verzweigt. Der Einfachheit halber ist jedoch eine gradlinige zweite peelbare Naht an einer Ecke des Behälters eingeschweißt, die einen kleinen Teil eines Flüssigkeitskompartimentes abtrennt. Die Flüssigkeit wird dabei soweit als möglich in das Flüssigkeitskompartiment zurückverdrängt. In einer bevorzugten Ausführung findet sich diese Trennaht an der Säurekompartimentseite.

Selbstverständlich muß in einem derartigen Fall gewährleistet sein, daß sich die Trennmittel zwischen den zu mischenden Flüsigkeitskompartimenten öffnen, bevor sich die Absperrung zum Auslaß hin öffnet. Dies wird im vorliegenden Ausführungsbeispiel zum einen dadurch erreicht, daß die Peelnähte unterschiedlich lang sind, zum anderen durch die Stärke der Verschweißung, die beispielsweise durch Druck- und Hitzevariabilität einstellbar ist.

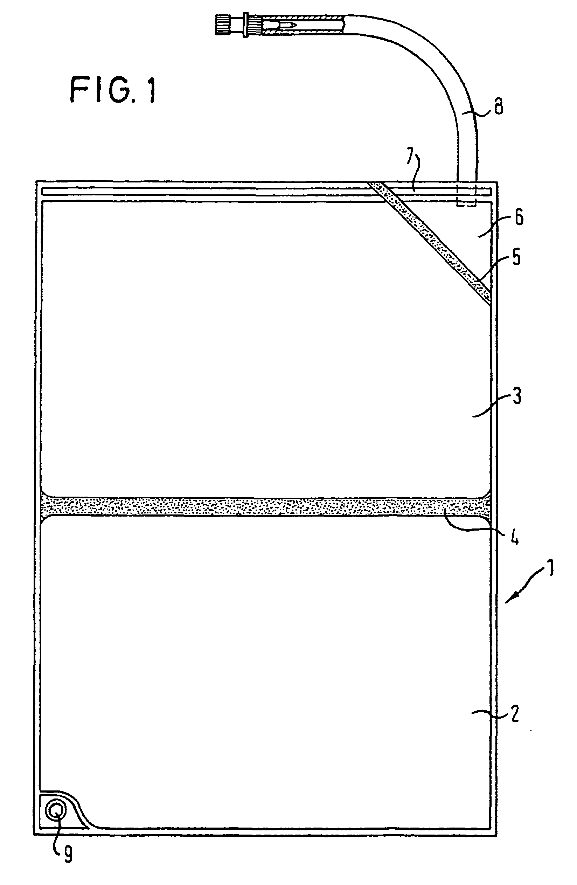

Figurenbeschreibung: In der Figur 1 ist beispielhaft ein Dreikammerbehälter (1) aus flexiblem polymeren Material aufgezeigt, der in seinem Umfang festverschweißt ist und drei Lösungskompartimente aufweist: Das erste Kompartiment (2) enthält ein Glucosekonzentrat, das zweite Kompartiment (3) ein Salzsäurekonzentrat. Zwischen beiden Lösungskompartimenten befindet sich ein peelbare Trennaht (4). Das Salzsäurekompartiment weist an seiner Außenkante eine weitere peelbare Trennaht (5) auf, die einen leeren Bereich (6) vom Lösungsbereich abtrennt. In der festverschweißten Umfangsnaht (7) ist ein Auslaß (8) dicht eingesetzt, im vorliegenden Fall ein Schlauchstück, das zu dem Mischsystem der Dialysemaschine führt. Vorzugsweise ist in der der Auslaßkante gegenüberliegenden Kante eine Aufhängevorrichtung (9) vorgesehen.

## Patentansprüche

1. Behälter zur Bereitstellung eines medizinischen Konzentrates, insbesondere für die Nierendialyse, bestehend aus mindestens zwei Kompartimenten und lösbaren Trennmitteln zwischen den Kompartimenten, wobei das erste Kompartiment mit einem ersten im wesentlichen glucosehaltigen Teilkonzentrat gefüllt ist, dessen Glucosegehalt über 10% liegt, und das zweite Kompartiment ein zweites Teilkonzentrat mit den Elektrolyten Calcium, Magnesium und Kalium in saurer Lösung enthält, wobei das elektrolythaltige Konzentrat freie Salzsäure enthält.

2. Behälter nach Anspruch 1, **dadurch gekennzeichnet, daß** das elektrolythaltige zweite Konzentrat mindestens 2% Salzsäure enthält.

3. Behälter nach Anspruch 1, **dadurch gekennzeichnet, daß** das elektrolythaltige zweite Teilkonzentrat 2,7% Salzsäure enthält.

4. Behälter nach Anspruch 1, **dadurch gekennzeichnet, daß** der Glucosegehalt des glucosehaltigen ersten Teilkonzentrates 20-30% ist.

5. Behälter nach Anspruch 1, **dadurch gekennzeichnet, daß** der Glucosegehalt des glucosehaltigen ersten Teilkonzentrates 27% ist.

6. Behälter nach Anspruch 1, **dadurch gekennzeichnet, daß** ein weiteres, im wesentliches leeres Kompartiment vorgesehen ist; das mit einem lösbaren Trennmittel von den anderen Kompartimenten getrennt ist.

7. Behälter nach Anspruch 4, **dadurch gekennzeichnet, daß** das dritte, leere Kompartiment einen Auslaß in seinem Randbereich aufweist.

8. Behälter nach Anspruch 1 bis 5, **dadurch gekennzeichnet, daß** die lösbaren Trennmittel peelbare Trennähte sind.

9. Behälter nach Anspruch 4 bis 6, **dadurch gekennzeichnet, daß** die Trennaht zum Abtrennen der Auslaßkammer die höchste Haftung von allen peelbaren Nähten aufweist.

10. Behälter nach Anspruch 8, **dadurch gekennzeichnet, daß** die Elektrolytgehalte zwischen 0 und 4M Natriumchlorid, bis 800mM Kaliumchlorid, 70 bis 400mM Calciumchlorid und 17,5 bis 200mM Magnesiumchlorid liegen.

## Claims

1. A container for the preparation of a medical concentrate, in particular for renal dialysis, comprising at least two compartments and releasable separating means between the compartments, wherein the first compartment is filled with a first substantially glucose-bearing partial concentrate whose glucose content is above 10% and the second compartment contains a second partial concentrate with the electrolytes calcium, magnesium and potassium in acid solution, wherein the electrolyte-bearing concentrate contains free hydrochloric acid.

2. A container according to claim 1 **characterised in that** the electrolyte-bearing second concentrate contains at least 2% hydrochloric acid.

3. A container according to claim 1 **characterised in that** the electrolyte-bearing second concentrate contains 2.7% hydrochloric acid.

4. A container according to claim 1 **characterised in that** the glucose content of the glucose-bearing first partial concentrate is 20 - 30%.

5. A container according to claim 1 **characterised in that** the glucose content of the glucose-bearing first partial concentrate is 27%.

6. A container according to claim 1 **characterised in that** there is provided a further, substantially empty compartment which is separated from the other compartments by a releasable separating means.

7. A container according to claim 4 **characterised in that** the third empty compartment has an outlet in its edge region.

8. A container according to claims 1 to 5 **characterised in that** the releasable separating means are peelable separating seams.

9. A container according to claims 4 to 6 **characterised in that** the separating seam for separating the outlet chamber has the highest adhesion of all peelable seams.

10. A container according to claim 8 **characterised in that** the electrolyte contents are between 0 and 4M sodium chloride, up to 800mM potassium chloride, 70 to 400mM calcium chloride and 17.5 to 200mM magnesium chloride.

## Revendications

1. Sac pour la préparation d'un concentré médical, en particulier pour la dialyse des reins, se composant d'au moins deux compartiments et de moyens détachables de séparation entre les compartiments et le premier compartiment est rempli d'un premier concentré partiel contenant essentiellement du glucose dont la teneur en glucose est supérieure à 10% et le second compartiment contient un second concentré partiel avec les électrolytes calcium, magnésium, et potassium en solution acide, le concentré contenant les électrolytes contenant de l'acide chlorhydrique libre.

2. Sac selon la revendication 1, **caractérisé en ce que** le second concentré contenant les électrolytes contient au moins 2% d'acide chlorhydrique.

3. Sac selon la revendication 1, **caractérisé en ce que** le second concentré partiel contenant les électrolytes contient 2,7% d'acide chlorhydrique.

4. Sac selon la revendication 1, **caractérisé en ce que** la teneur en glucose du premier concentré partiel contenant du glucose est de 20-30%.

5. Sac selon la revendication 1, **caractérisé en ce que** la teneur en glucose du premier concentré partiel contenant du glucose est de 27%.

6. Sac selon la revendication 1, **caractérisé en ce qu'**un autre compartiment, essentiellement vide, est prévu, qui est séparé par un moyen de séparation détachable des autres compartiments.

7. Sac selon la revendication 4, **caractérisé en ce que** le troisième compartiment vide présente une sortie dans sa zone de bordure.

8. Sac selon la revendication 1 à 5, **caractérisé en ce que** le moyen de séparation détachable sont des soudures de séparation écaillables.

9. Sac selon la revendication 4 à 6, **caractérisé en ce que** la soudure de séparation pour la séparation de la chambre de sortie présente la plus haute retenue de toutes les soudures écaillables.

10. Sac selon la revendication 8, **caractérisé en ce que** les teneurs en électrolytes sont comprises entre 0 et 4 M de chlorure de sodium, jusqu'à 800mM de chlorure de potassium, 70 à 400mM de chlorure de calcium et 17,5 à 200mM de chlorure de magnésium.
